# EUROPEAN PATENT APPLICATION

(11) **EP 4 005 489 A1**
(43) Date of publication of application: **01.06.2022**
(21) Application number: 20847432.0
(22) Date of filing: 20.07.2020
(51) Int. Cl.: A61B 7/04

(54) **STETHOSCOPE**

(30) Priority: 26.07.2019 JP 2019138274
(71) Applicant: FUJIFILM Corporation, Tokyo 106-8620 (JP)
(72) Inventor: SASAKI, Tsutomu, Ashigarakami-gun, Kanagawa 258-8538 (JP)
(74) Representative: Kudlek, Franz Thomas
(86) International application number: PCT/JP2020/028079
(87) International publication number: WO 2021/020201

(57) **Abstract**

Provided is a stethoscope including: a support base having a first contact surface that comes into contact with an object to be measured, in which each of three first connection sides that are at least parts of sides defining the first contact surface is at least a part of each side of a triangle including the first contact surface; three elastic members having elasticity each of which has a second contact surface that has an obtuse angle formed with the first contact surface and that comes into contact with the object to be measured, the three elastic members being connected to each of the first connection sides in a second connection side having the same length as the first connection side out of sides defining the second contact surface; and three electrocardiographic electrodes that are disposed on each of the second contact surfaces and detect cardiac electrical activity of the object to be measured.

## Description

### Technical Field

The present disclosure relates to a stethoscope. In particular, the present disclosure relates to an electronic stethoscope that comprises an electrocardiographic electrode which detects cardiac electrical activity of an object to be measured and that can output a detected sound and cardiac electrical activity, as data.

### Background Art

Hitherto, for a stethoscope that can amplify and listen to sounds, such as a heart sound and a blood flow sound, which are generated inside a living body (hereinafter, referred to as a body sound) and that comprises an electrocardiographic electrode which detects cardiac electrical activity of an object to be measured, the following techniques have been known.

For example, JP2012-55354A describes an electric stethoscope including a microphone that is used to record a heart sound waveform, a fixed electrode that is used to record an electrocardiogram, and a movable electrode attached to a rotating arm.

JP2017-170112A describes a stethoscope including a stethoscope unit consisting of a chest piece and a microphone, and an electrode for electrocardiography of which a surface coming into contact with a human body is formed on the same side as a contact surface of the chest piece with the human body or on a side slightly away from the human body than the contact surface.

JP2012-55354A describes a diagnostic apparatus including a hearing sound detection unit that detects a body sound, a tube of which one end is inserted into the hearing sound detection unit and the other end is used to auscultate the body sound, an electrocardiogram detection unit that is provided in the hearing sound detection unit and detects potential associated with heartbeat, and a control unit that converts the potential detected by the electrocardiogram detection unit into a radio signal and transmits the converted signal.

### SUMMARY OF INVENTION

### Technical Problem

Incidentally, an electrocardiographic electrode may include three electrocardiographic electrodes having different functions. In this case, each of the electrocardiographic electrodes has a predetermined area, and the three electrocardiographic electrodes are disposed sufficiently apart from each other, so that a myoelectric influence of an object to be measured can be eliminated, and cardiac electrical activity can be detected with a high signal to noise ratio (SN ratio).

However, in the stethoscopes described in JP2012-55354A, JP2017-170112A, and JP2012-55354A, in a case where the area of the electrocardiographic electrode is increased and the three electrocardiographic electrodes are spaced apart from each other, the size of the stethoscope itself becomes large, and the operability of the user decreases.

The present disclosure provides a stethoscope capable of detecting cardiac electrical activity with a high SN ratio while restraining a decrease in operability of the user.

### Solution to Problem

There is provided a stethoscope according to a first aspect of the present disclosure comprising: a support base having a first contact surface that comes into contact with an object to be measured, in which each of three first connection sides that are at least parts of sides defining the first contact surface is at least a part of each side of a triangle including the first contact surface; three elastic members having elasticity each of which has a second contact surface that has an obtuse angle formed with the first contact surface and that comes into contact with the object to be measured, the three elastic members being connected to each of the first connection sides in a second connection side having the same length as the first connection side out of sides defining the second contact surface; and three electrocardiographic electrodes that are disposed on each of the second contact surfaces and detect cardiac electrical activity of the object to be measured.

In a second aspect of the present disclosure, in the above-described first aspect, the support base may have an opening portion on the first contact surface, and a detection unit that is supported by the support base in a state in which a part of a surface of the detection unit is exposed from the opening portion and that detects a vibration caused by a sound which is generated from the object to be measured may further be provided.

In a third aspect of the present disclosure, in the above-described aspects, each of the second contact surfaces may have an area of 100 mm² or more.

In a fourth aspect of the present disclosure, in the above-described aspects, the elastic member may have a thickness of 0.5 mm or more and 50 mm or less.

In a fifth aspect of the present disclosure, in the above-described aspects, the elastic member may have a Young's modulus of 0.2 MPa or more and 50 MPa or less.

In a sixth aspect of the present disclosure, in the above-described aspects, the elastic member may contain any one of an elastomer material, a silicone resin, a silicone rubber, a urethane rubber, a natural rubber, a styrene-butadiene rubber, a chloroprene rubber, an acrylonitrile rubber, a butyl rubber, an ethylene propylene rubber, a fluoro-rubber, or a chlorosulfonated polyethylene rubber.

In a seventh aspect of the present disclosure, in the above-described aspects, the support base may be formed of a member harder than the elastic member.

In an eighth aspect of the present disclosure, in the above-described aspects, the elastic member may have a shape that is tapered in a direction away from the second connection side.

In a ninth aspect of the present disclosure, in the above-described aspects, the first contact surface and each of the second contact surfaces may be formed so as to extend in the same plane, in a case where a pressing force with which the first contact surface is pressed against the object to be measured is applied.

In a tenth aspect of the present disclosure, in the above-described aspects, the three electrocardiographic electrodes may be a positive electrode, a negative electrode, and a reference electrode that measures a reference level.

### Advantageous Effects of Invention

According to the above-described aspects, the stethoscope of the present disclosure can detect cardiac electrical activity with a high SN ratio while restraining a decrease in the operability of the user.

### BRIEF DESCRIPTION OF DRAWINGS

Fig. 1 is a perspective view showing a rear side of a stethoscope according to a first exemplary embodiment.
Fig. 2 is a perspective view showing a side of a contact surface of the stethoscope according to the first exemplary embodiment.
Fig. 3 is a diagram showing the contact surface of the stethoscope according to the first exemplary embodiment.
Fig. 4 is a cross-sectional view taken along a line A-A of Fig. 3.
Fig. 5 is a cross-sectional view taken along the line A-A of Fig. 3 in a case where the stethoscope according to the first exemplary embodiment is pressed against a living body.
Fig. 6 is a view of another stethoscope filled with a gas that supports a piezoelectric film.
Fig. 7 is a schematic view showing configurations of the piezoelectric film and a protective layer.
Fig. 8 is a diagram illustrating a polarization action of the piezoelectric layer.
Fig. 9 is a graph in which an SN ratio is measured in a case where an acoustic impedance of the protective layer is changed.
Fig. 10 is a schematic view showing a configuration of another protective layer formed of a plurality of layers.
Fig. 11 is a schematic view showing a configuration of another protective layer of which a surface consists of a hydrophobic material.
Fig. 12 is a block diagram showing a configuration of the stethoscope according to the first exemplary embodiment.
Fig. 13 is a block diagram showing a configuration of an output unit according to the first exemplary embodiment.
Fig. 14 is a cross-sectional view taken along the line A-A of Fig. 3 relating to a stethoscope according to a second exemplary embodiment.
Fig. 15 is a cross-sectional view taken along the line A-A of Fig. 3 in a case where the stethoscope according to the second exemplary embodiment is pressed against the living body.

### DESCRIPTION OF EMBODIMENTS

Hereinafter, exemplary embodiments of the technique of the present disclosure will be described in detail with reference to the drawings. Hereinafter, description will be made by using a living body 12 as an example of an object to be measured and a body sound as an example of a sound that is generated from the object to be measured. Examples of the body sound include heartbeat, respiratory sounds, blood flow sounds, and intestinal sounds. Hereinafter, the piezoelectric film 30 is used as an example of the detection unit.

### [First Exemplary Embodiment]

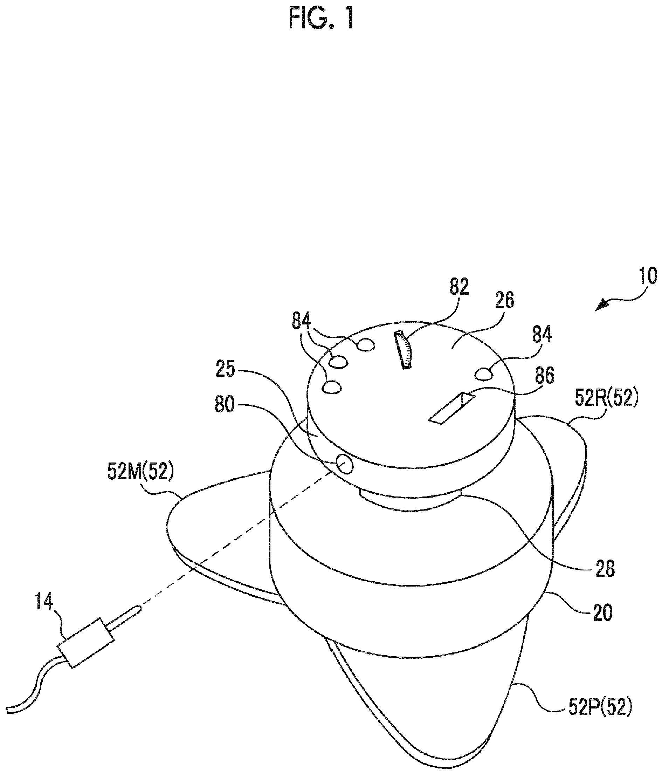

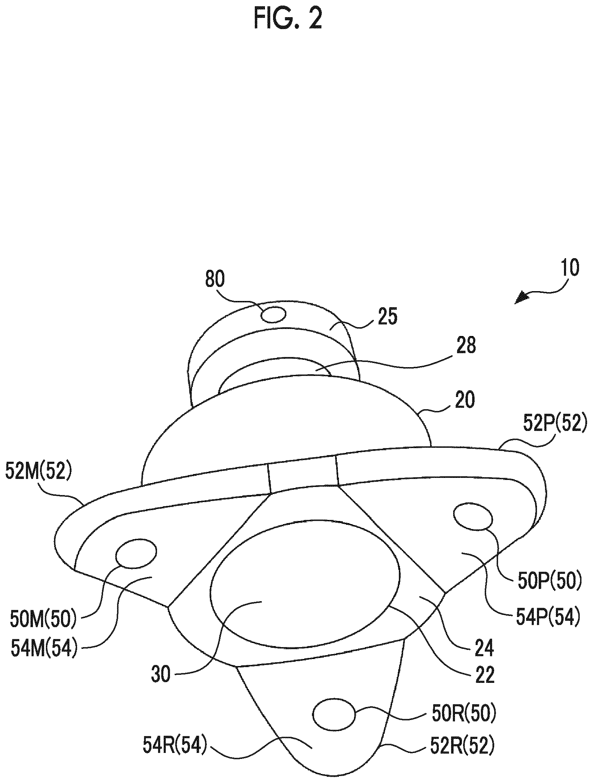

First, a configuration of a stethoscope 10 according to the present exemplary embodiment will be described with reference to Figs. 1 to 3. As shown in Figs. 1 to 3, the stethoscope 10 comprises a support base 20, a piezoelectric film 30, and elastic members 52P, 52M, and 52R in which a positive electrode 50P, a negative electrode 50M, and a reference electrode 50R are disposed, respectively, as an example of an electrocardiographic electrode.

The support base 20 has an opening portion 22, a first contact surface 24 that extends around the opening portion 22 and that comes into contact with the living body 12, a rear surface 26 provided on a side opposite to the first contact surface 24, and a side surface 25 that is connected to the first contact surface 24 and the rear surface 26. Further, the support base 20 has a neck portion 28 having a diameter smaller than that of each of the first contact surface 24 and the rear surface 26, in the side surface 25. Further, the support base 20 is formed of a member harder than the elastic member 52, which will be described later.

The support base 20 has an output terminal 80 on the side surface 25. The output terminal 80 is a terminal from which a signal indicating a body sound is output (adjustment signal S3, which will be described later). The output terminal 80 includes, for example, an earphone jack to which the terminal of an earphone 14 that is used to listen to the body sound acquired by the stethoscope 10 is connected. The output terminal 80 is preferably disposed closer to the rear surface 26 than the neck portion 28 of the side surface 25. With such a configuration, the terminal of the earphone 14 connected to the output terminal 80 does not interfere with a hand of a user in a case where the user holds the neck portion 28 by the hand, so that the operability can be improved.

The support base 20 has an input unit 82, a display unit 84, and a charging terminal 86 that is used to charge a battery 96, which will be described later, on the rear surface 26. The input unit 82 is a portion in which an operation for adjusting a volume level of the body sound heard by using the earphone 14 is performed. The input unit 82 includes, for example, a dial-type input component. With the input unit 82 made to be a dial type, it is possible to restrain erroneous operation by the user as compared with the button-type. The display unit 84 includes, for example, an LED light and displays a remaining amount of the battery 96 and the like.

The elastic members 52P, 52M, and 52R have second contact surfaces 54P, 54M, and 54R that come into contact with the living body 12, respectively, and are members each of which has elasticity and is connected to the periphery of the support base 20. Hereinafter, in a case where the elastic members 52P, 52M, and 52R are not distinguished from each other, the elastic members 52P, 52M, and 52R are referred to as an elastic member 52. In a case where the second contact surfaces 54P, 54M, and 54R are not distinguished from each other, the second contact surfaces 54P, 54M, and 54R are referred to as a second contact surface 54.

The elastic member 52 may contain, for example, any one of an elastomer material, a silicone resin, a silicone rubber, a urethane rubber, a natural rubber, a styrene-butadiene rubber, a chloroprene rubber, an acrylonitrile rubber, a butyl rubber, an ethylene propylene rubber, a fluoro-rubber, or a chlorosulfonated polyethylene rubber. The elastic member 52 containing the materials has higher durability and lower flexibility as the thickness increases and the Young's modulus increases. Low flexibility may cause discomfort due to the hardness in a case where the elastic member 52 is pressed against the living body 12.

Therefore, the elastic member 52 preferably has a thickness of 0.5 mm or more and 50 mm or less. The thickness is more preferably 3 mm or more and 30 mm or less, and most preferably 5 mm or more and 20 mm or less. Further, the elastic member 52 preferably has a Young's modulus of 0.2 MPa or more and 50 MPa or less. The elastic member 52 more preferably has a Young's modulus of 0.2 MPa or more and 10 MPa or less. With the elastic member 52 made to have the thickness and Young's modulus in the above-described ranges, it is possible to obtain a member having durability and flexibility suitable for use as a stethoscope.

The positive electrode 50P, the negative electrode 50M, and the reference electrode 50R that is used to measure a reference level are disposed on the second contact surfaces 54P, 54M, and 54R, respectively. That is, each of the three electrocardiographic electrodes of the positive electrode 50P, the negative electrode 50M, and the reference electrode 50R is disposed around the piezoelectric film 30. Hereinafter, in a case where the positive electrode 50P, the negative electrode 50M, and the reference electrode 50R are not particularly distinguished from each other, the positive electrode 50P, the negative electrode 50M, and the reference electrode 50R are referred to as an electrocardiographic electrode 50.

The electrocardiographic electrode 50 is preferably attachable to and detachable from the elastic member 52. For example, the electrocardiographic electrode 50 and the elastic member 52 may be provided with a pair of coupling members (for example, a snap coupling member). Further, for example, a surface of at least one of the electrocardiographic electrode 50 or the elastic member 52, which comes into contact with each other, may be provided with an attachable and detachable adhesive member. With the electrocardiographic electrode 50 made attachable and detachable, the electrocardiographic electrode 50 can be replaced as appropriate.

As the electrocardiographic electrode 50, a commercially available disposable type electrocardiographic electrode may be used. The surface of the electrocardiographic electrode 50, which comes into contact with the living body 12, preferably has adhesiveness. Specifically, an adhesive strength measured by the following measuring method is preferably 0.25 N/mm (3 N/12 mm) or less.

### [180° Peeling Test]

One end of a test piece having a width of 12 mm was stuck to an end of a test plate, and immediately, while a pressure was applied at a speed of 1 mm/s by using a pressing roller, the test piece was stuck to the test plate such that the other end side of the test piece remains as a pulling margin. The test plate was set in a testing machine (AGS-X (manufactured by Shimadzu Corporation) or ZTA (manufactured by IMADA Co., Ltd.)), the test piece was peeled off from the test plate at a speed of 10 mm/s, and an average value of measured values (N) from after the measured value was stabilized to the end of peeling was calculated and a value of adhesive strength was obtained.

For the cardiac electrical activity that is detected by the electrocardiographic electrode 50 and the body sound that is detected by the piezoelectric film 30 (details will be described later), in a case where the stethoscope 10 is in contact with the living body 12 without moving for a certain period of time, the cardiac electrical activity and the body sound are detected. Therefore, with the electrocardiographic electrode 50 having adhesiveness on the surface that comes into contact with the living body 12, the stethoscope 10 can be fixed to the living body 12, and the stethoscope 10 can be restrained from moving easily. That is, with the electrocardiographic electrode 50 having adhesiveness, it is possible to restrain a decrease in the detection efficiency of the cardiac electrical activity and the body sound in a case where the stethoscope 10 is brought into contact with the living body 12.

In order for the electrocardiographic electrode 50 to detect the cardiac electrical activity of the living body 12 with sufficient accuracy, it is preferable that the electrocardiographic electrode 50 has a predetermined area. Therefore, in the elastic member 52 in which the electrocardiographic electrode 50 is disposed, the area of the second contact surface 54 is preferably 100 mm² or more. With the second contact surface 54 made to have the area in the above-described range, the electrocardiographic electrode 50 having a sufficient area can be disposed, and the cardiac electrical activity can be detected with sufficient accuracy.

Here, a position of the output terminal 80 according to the present exemplary embodiment will be described. In a case where the electrocardiographic electrode 50 is brought into contact with the living body 12, a direction of an electrocardiographic signal S5 (details will be described later) that is output varies depending on a positional relationship between the heart of the living body 12, and the positive electrode 50P, the negative electrode 50M, and the reference electrode 50R. In order to output the electrocardiographic signal S5 in a specified direction, it is assumed that the direction of the electrocardiographic electrode 50 with respect to the living body 12 is required to be determined so that, for example, the reference electrode 50R faces a side of a head of the living body 12 and the positive electrode 50P and the negative electrode 50M face a side of a leg of the living body 12. Therefore, it is desired that the user can easily determine which functions the three electrocardiographic electrodes 50 have.

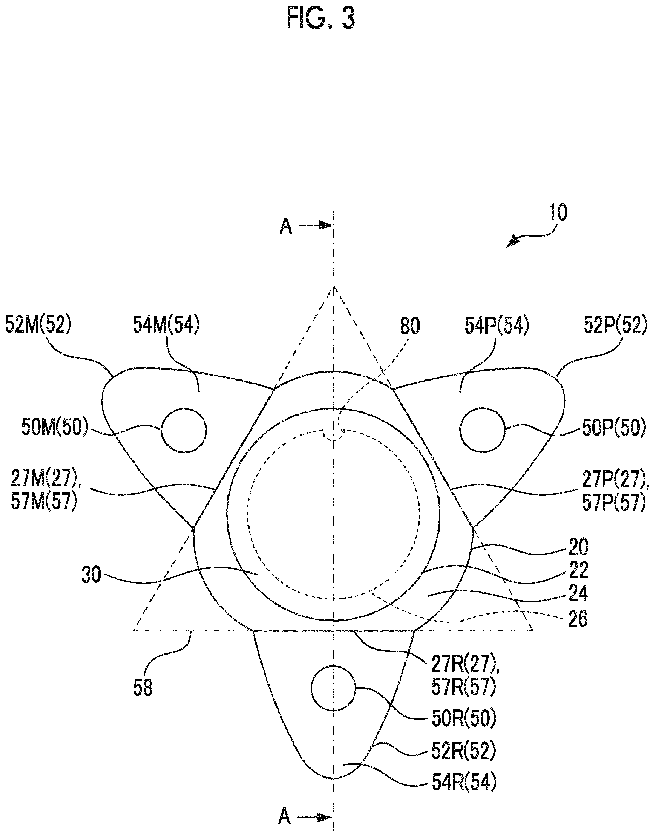

As shown in Fig. 3, the output terminal 80 is disposed on an axis A-A passing through the center of one predetermined electrocardiographic electrode 50 out of the three electrocardiographic electrodes 50 and the center of the piezoelectric film 30. In the present exemplary embodiment, the electrocardiographic electrode 50 disposed on the axis A-A is the reference electrode 50R. Further, the positive electrode 50P and the negative electrode 50M are disposed around the piezoelectric film 30 at positions symmetrical with respect to the axis A-A, respectively.

With such a configuration, the user can easily determine which electrocardiographic electrode 50 is the reference electrode 50R on the basis of the position of the output terminal 80. Therefore, the operability of the user can be improved.

As shown in Fig. 3, the disposition of the output terminal 80 is not limited to the position which faces the reference electrode 50R with the piezoelectric film 30 interposed therebetween. For example, the output terminal 80 may be disposed on the axis A-A and at a position closest to the reference electrode 50R.

Further, the electrocardiographic electrode 50 disposed on the axis A-A is not limited to the reference electrode 50R. The three electrocardiographic electrodes 50 need only be distinguished from each other on the basis of the positional relationship with the output terminal 80, and the positive electrode 50P and the negative electrode 50M may be used as the electrocardiographic electrode 50 that is disposed on the axis A-A.

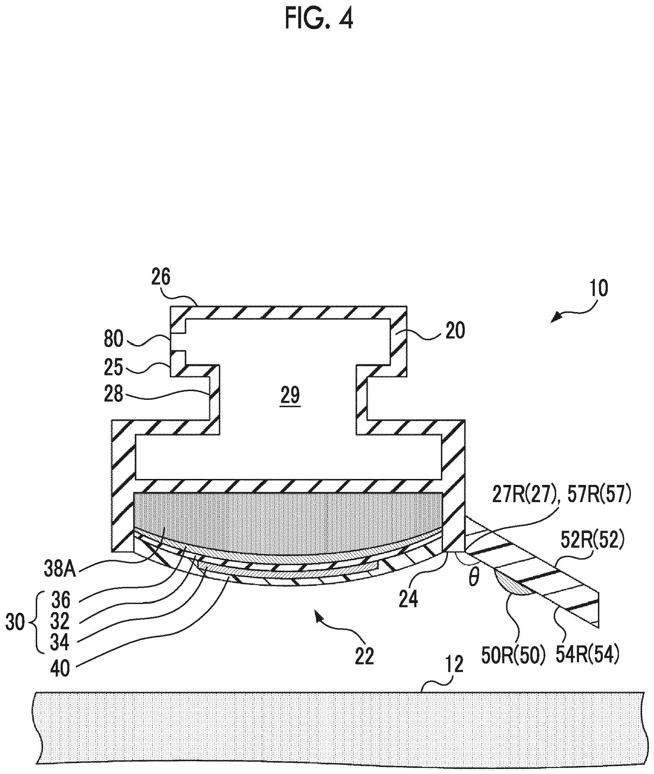

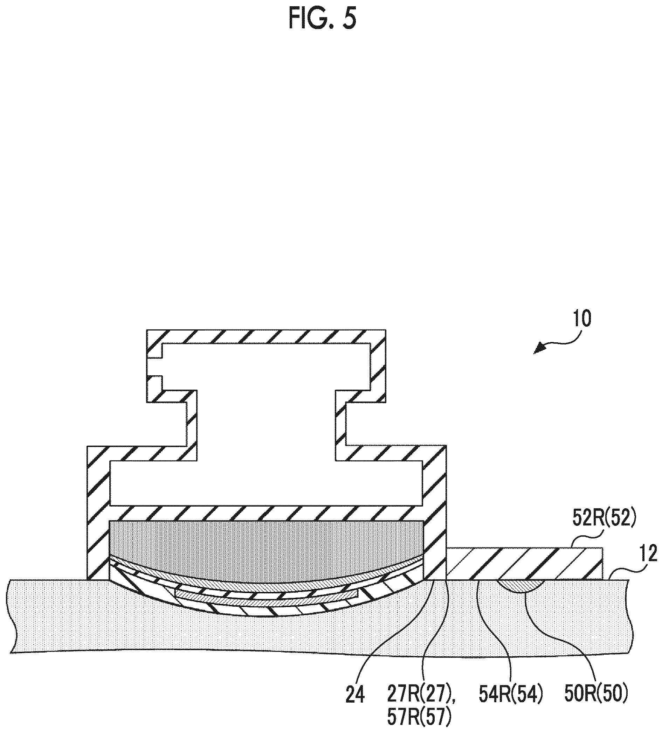

Next, a connection relationship between the support base 20 and the elastic member 52 according to the present exemplary embodiment will be described with reference to Figs. 3 to 5. Figs. 4 and 5 are cross-sectional views taken along the line A-A of Fig. 3, and Fig. 4 shows a case where the stethoscope 10 is not pressed against the living body 12, and Fig. 5 shows a case where the stethoscope 10 is pressed against the living body 12.

As described above, in order for the electrocardiographic electrode 50 to detect the cardiac electrical activity of the living body 12 with sufficient accuracy, the electrocardiographic electrode 50 needs to have a predetermined area. In addition, with the three electrocardiographic electrodes 50 disposed sufficiently apart from each other, a myoelectric influence of the living body 12 can be eliminated, and the cardiac electrical activity can be detected with a high SN ratio.

As shown in Fig. 3, the support base 20 has three first connection sides 27P, 27M, and 27R that are connected to the elastic members 52P, 52M, and 52R, respectively, as sides defining the first contact surface 24. Each of the first connection sides 27P, 27M, and 27R forms at least a part of each side of a triangle 58 including the first contact surface 24. Hereinafter, in a case where the first connection sides 27P, 27M, and 27R are not particularly distinguished from each other, the first connection sides 27P, 27M, and 27R are referred to as a first connection side 27.

The elastic member 52P has a second connection side 57P that is connected to the first connection side 27P of the support base 20, as a side defining the second contact surface 54P. The length of the second connection side 57P is equal to the length of the first connection side 27P. Similarly, the elastic member 52M has a second connection side 57M that is connected to the first connection side 27M of the support base 20, as a side defining the second contact surface 54M. The length of the second connection side 57M is equal to the length of the first connection side 27M. Similarly, the elastic member 52R has a second connection side 57R that is connected to the first connection side 27R of the support base 20, as a side defining the second contact surface 54R. The length of the second connection side 57R is equal to the length of the first connection side 27R. Hereinafter, in a case where the second connection sides 57P, 57M, and 57R are not particularly distinguished from each other, the second connection sides 57P, 57M, and 57R are referred to as a second connection side 57. Here, in a case where the length of the second connection side 57 is "equal" to the length of the first connection side 27, the term "equal" is not limited to a case of being completely equal thereto, and the length need only be a length in which the second connection side 57 is stably connected to the first connection side 27, for example, may be a length having a difference of about ± 10%. Further, the elastic member 52 has a shape that is tapered in a direction away from the second connection side 57.

As shown in Fig. 4, in a state in which the first contact surface 24 is not pressed against the living body 12, an angle θ of the elastic member 52R formed by the second contact surface 54R and the first contact surface 24 is an obtuse angle. The same applies to the elastic members 52P and 52M.

As shown in Fig. 5, the first contact surface 24 and the second contact surface 54R of the elastic member 52R are formed so as to extend in the same plane, in a case where a pressing force with which the first contact surface 24 of the support base 20 is pressed against the living body 12 is applied to the stethoscope 10. The same applies to the elastic members 52P and 52M.

As described above, the elastic member 52 has a Young's modulus of 0.2 MPa or more and 50 MPa or less, and the support base 20 is formed of a member harder than the elastic member 52. Therefore, the elastic member 52 can be bent such that the first contact surface 24 and the second contact surface 54 extend in the same plane, with the first connection side 27 and the second connection side 57 as a starting point. In this case, since a bias force acts in a direction of pressing the second contact surface 54 against the living body 12, the electrocardiographic electrode 50 and the living body 12 can be brought into close contact with each other, and the cardiac electrical activity can be detected with a high SN ratio.

The triangle 58 is not limited to an equilateral triangle as shown in Fig. 3. For example, the triangle 58 may be an isosceles triangle in which the reference electrode 50R is disposed on the base and the positive electrode 50P and the negative electrode 50M are disposed on the equal sides, respectively.

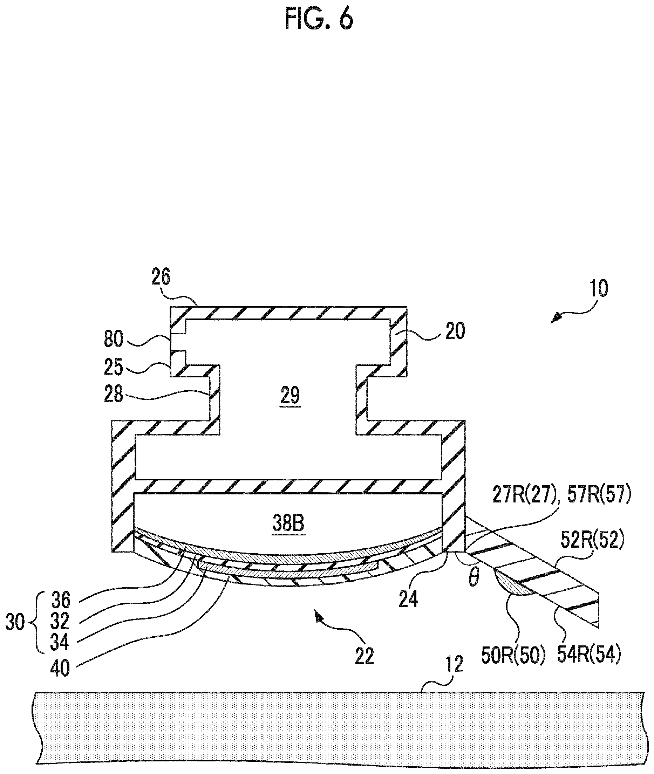

Next, the configurations of the piezoelectric film 30 and the protective layer 40 according to the present exemplary embodiment will be described with reference to Figs. 4 and 6 to 11. Fig. 6 is an enlarged schematic view of parts of the piezoelectric film 30 and the protective layer 40 in order to show the configurations of the piezoelectric film 30 and the protective layer 40.

As shown in Fig. 4, the piezoelectric film 30 is supported by the support base 20 in a state in which the surface exposed from the opening portion 22 is convexly curved. Further, the surface of the piezoelectric film 30, which is exposed from the opening portion 22, protrudes with respect to the first contact surface 24.

Since the surface of the piezoelectric film 30, which is exposed from the opening portion 22, is convexly curved, it is possible to increase the expansion and contraction of the piezoelectric film 30 in the in-plane direction as compared with a case where the piezoelectric film 30 is provided in a flat shape. That is, with the piezoelectric film 30 supported by the support base 20 in a state in which the surface exposed from the opening portion 22 is convexly curved, it is possible to increase the amplitude of the voltage that is detected by the piezoelectric film 30 and a sound can be collected with a high SN ratio.

The piezoelectric film 30 may be directly connected to a part of the support base 20, or may be connected to the support base 20 via another member such as an adhesive. Further, the piezoelectric film 30 has elasticity and flexibility that a crack does not occur in a case where the piezoelectric film 30 is pressed against the living body 12.

A space between the piezoelectric film 30 and the support base 20 is filled with a cushioning material 38A, and the piezoelectric film 30 is supported by the cushioning material 38A so as to be convexly curved. The cushioning material 38A has appropriate elasticity, and supports the piezoelectric film 30 and imparts a constant mechanical bias to the entire surface of the piezoelectric film 30. As a result, the vibration in the thickness direction, which is generated in the piezoelectric film 30, can be converted into an expansion/contraction motion in the in-plane direction of the piezoelectric film 30, and the electric charge generation efficiency can be improved. Further, the filling density of the cushioning material 38A is changed, so that a stethoscope 10 having an appropriate repulsive force can be realized.

The material of the cushioning material 38Aneed only be a material that has appropriate elasticity, and that is suitably deformed without hindering the vibration of the piezoelectric film 30. Specifically, for example, Alpha GEL (registered trademark) (manufactured by Taica Corporation) using silicone as a main raw material, wool felt containing polyester fibers such as rayon and polyethylene terephthalate (PET), a fiber material such as glass wool, and a foaming material such as polyurethane are preferably used. As shown in Fig. 6, the space may be filled with a gas 38B instead of the cushioning material 38A.

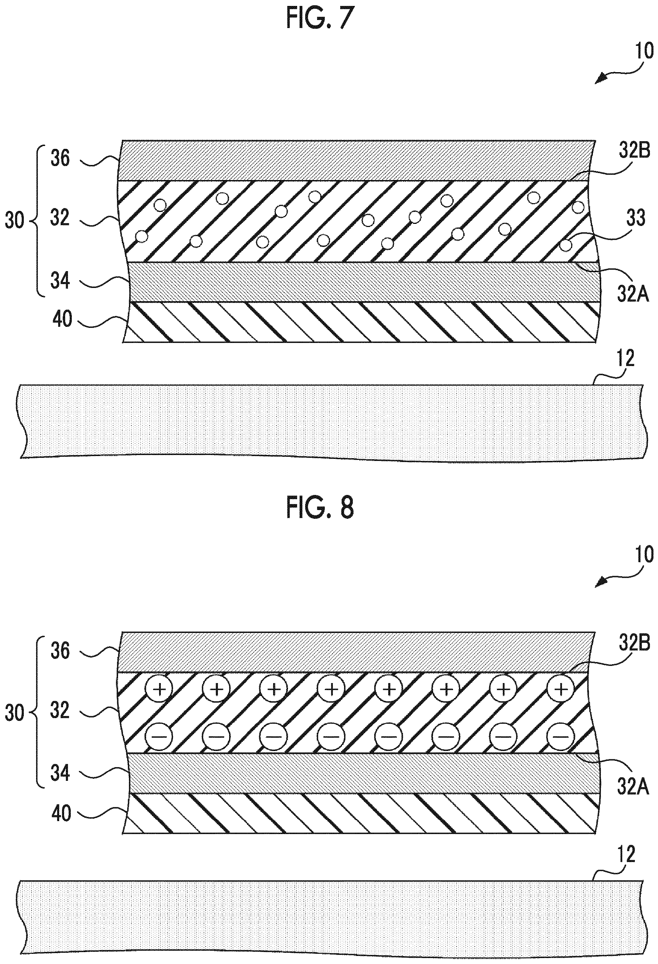

As shown in Fig. 7, the piezoelectric film 30 comprises a piezoelectric layer 32 that has a first main surface 32A which is a main surface on a side of the living body 12 and a second main surface 32B which is a main surface opposite to the side of the living body 12, as two main surfaces facing each other, a first electrode 34 provided on the first main surface 32A, and a second electrode 36 provided on the second main surface 32B.

The piezoelectric layer 32 generates the expansion and contraction in the in-plane direction in response to the body sound that is emitted from the living body 12, and generates a voltage between the first electrode 34 and the second electrode 36 in response to the expansion and contraction in the in-plane direction. In the present exemplary embodiment, as the piezoelectric layer 32, a polymer-based piezoelectric composite material in which piezoelectric particles 33 are dispersed in a matrix consisting of a polymer material may be used. The piezoelectric particles 33 may be uniformly dispersed with regularity, or may be irregularly dispersed, in the matrix.

The matrix is preferably a polymer material having viscoelasticity at a room temperature, such as cyanoethylated polyvinyl alcohol (cyanoethylated PVA), polyvinyl acetate, polyvinylidene chloride core acrylonitrile, a polystyrene-vinyl polyisoprene block copolymer, polyvinyl methyl ketone, and polybutyl methacrylate.

The piezoelectric particle 33 is a particle of a piezoelectric material, and is preferably a ceramic particle having a perovskite-type crystal structure. Examples of the piezoelectric particle include lead zirconate titanate, lead lanthanum zirconate titanate, barium titanate, and a solid solution of barium titanate and bismuth ferrite.

As shown in Fig. 8, the piezoelectric layer 32 having such a configuration causes dielectric polarization in the thickness direction of the piezoelectric layer 32. In a case of the piezoelectric layer 32 that causes dielectric polarization in this way, it is preferable that the piezoelectric layer 32 is disposed such that a positive electric charge is generated on a side of a second main surface 32B and a negative electric charge is generated on a side of the first main surface 32A. It is known that the living body 12 is usually positively charged in many cases. Therefore, with the piezoelectric layer 32 disposed such that a negative electric charge is generated on the side of the first main surface 32A which is a surface on the side which comes into contact with the living body 12, the body sound can be efficiently detected.

As the piezoelectric layer 32, an organic piezoelectric film such as polyvinylidene fluoride (PVDF), vinylidene-ethylene trifluoride copolymer (P(VDF-TrFE)), or polylactic acid may be used. Alternatively, as the piezoelectric layer 32, an organic material such as a polymer electret material containing, as a main component, a polymer described in JP2018-191394A, JP2014-233688A, and JP2017-12270A may be used. Examples of the organic material include polyimide, polytetrafluoroethylene, polypropylene, Teflon (registered trademark) such as polytetrafluoroethylene (PTFE) (tetrafluoride), a tetrafluoroethylene-perfluoroalkyl vinyl ether copolymer (PFA), a tetrafluoroethylene-hexafluoropropylene copolymer (FEP) (tetra-hexafluoride), and an amorphous fluoropolymer (AF), polyethylene, and cycloolefin polymers (COCs).

However, in a case where PVDF is used as the piezoelectric layer 32 instead of the polymer-based piezoelectric composite material, dielectric polarization occurs in the in-plane direction. In this case, the SN ratio may decrease as compared with the polymer-based piezoelectric composite material in which dielectric polarization occurs in the thickness direction.

The first electrode 34 and the second electrode 36 detect the expansion and contraction in the in-plane direction, which is generated in the piezoelectric layer 32, as a voltage. The thickness of each of the first electrode 34 and the second electrode 36 is not particularly limited, but is preferably thin in order to ensure the flexibility of the piezoelectric film 30, and is preferably 1 µm or less, for example. The thicknesses of the first electrode 34 and the second electrode 36 may be the same or different from each other.

It is preferable that the material of the first electrode 34 and the second electrode 36 is a thin film of copper (Cu) or aluminum (Al) formed by vacuum deposition in order to ensure the flexibility of the piezoelectric film 30, a conductive polymer, and the like.

Various conductors may be used as the material of the first electrode 34 and the second electrode 36. For example, C, Pd, Fe, Sn, Ni, Pt, Au, Ag, Cr, and Mo, and an alloy thereof may be used. Alternatively, a transparent conductive film such as indium tin oxide (ITO), indium zinc oxide (IZO), tin oxide, and zinc oxide may be used. Alternatively, an organic conductor such as a conductive polymer may be used. A forming method of the electrode is also not particularly limited, and various known methods, for example, film formation by a vapor deposition method (vacuum film forming method) such as vacuum deposition and sputtering, screen printing, and a method of pasting a foil formed of the above-described materials may be used.

The size of at least one of the first electrode 34 or the second electrode 36 may be smaller than that of the piezoelectric layer 32. In particular, as shown in Fig. 4, the first electrode 34 is preferably provided only in a central portion of the first main surface 32A. In addition to the function of detecting the body sound that is emitted from the living body 12, the first electrode 34 may function as an antenna and take in electromagnetic noise from an outside. In order to restrain the first electrode 34 from taking in electromagnetic noise, it is preferable to reduce the size of the first electrode 34 within a range in which it is difficult to take in electromagnetic noise and a body sound can be sufficiently detected. With the first electrode 34 provided only in the central portion of the first main surface 32A that comes into contact with the living body 12, it is possible to restrain the first electrode 34 from taking in electromagnetic noise and a sound can be collected with a high SN ratio.

Since the surface of the piezoelectric film 30, which is exposed from the opening portion 22, is convexly curved and protrudes with respect to the first contact surface 24, the piezoelectric film 30 is easily damaged in a case where the piezoelectric film 30 is brought into direct contact with the living body 12, as compared with the case the piezoelectric film 30 is provided in a flat shape. Therefore, it is preferable to dispose the protective layer 40 on the surface of the piezoelectric film 30 on the side that comes into contact with the living body 12. With the protective layer 40 disposed on the surface of the piezoelectric film 30 on the side that comes into contact with the living body 12, damage to the piezoelectric film 30 can be prevented.

Further, in a case where the piezoelectric film 30 detects the vibration of the living body 12, a large difference in acoustic impedance (unit: MRayls = kg/m²s) therebetween, which is a value intrinsic to each substance, leads to sound reflection, so that the detection efficiency of a body sound decreases. That is, the ratio of noise increases and the SN ratio decreases. Therefore, in order to alleviate the difference in acoustic impedance between the living body 12 and the piezoelectric film 30, the protective layer 40 preferably has an acoustic impedance between the acoustic impedance of the living body 12 and the acoustic impedance of the piezoelectric film 30.

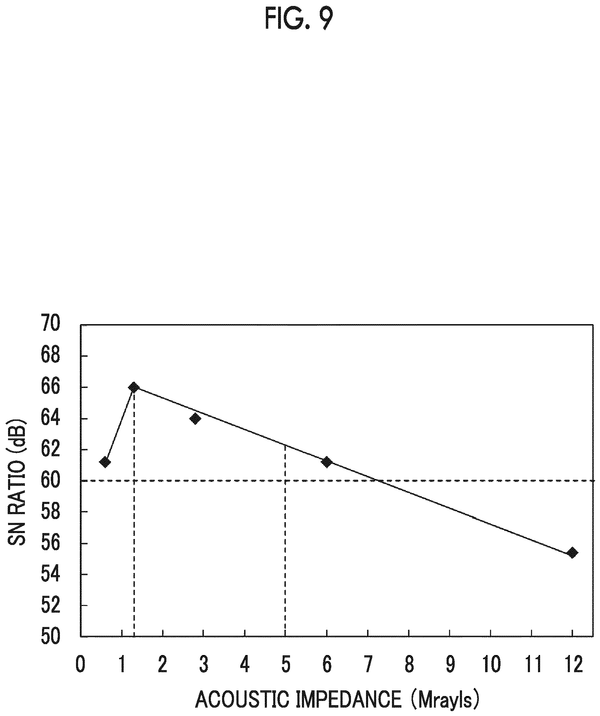

Fig. 9 is a graph in which the SN ratio of a sound signal S2 (details will be described later) that is output from the stethoscope 10 is measured in a case where the acoustic impedance of the protective layer 40 is changed. In Fig. 9, the horizontal axis represents the acoustic impedance (MRayls) of the protective layer 40, and the vertical axis represents the SN ratio (dB) of the sound signal S2.

The acoustic impedance of the living body 12 is known to be 1.3 to 1.5 MRayls, and the acoustic impedance of the piezoelectric film 30 is 5.0 to 10.0 MRayls. In a case where the protective layer 40 has an acoustic impedance of 1.3 MRayls or more and 5.0 MRayls or less between the acoustic impedance of the living body 12 and the acoustic impedance of the piezoelectric film 30, as shown in Fig. 9, the SN ratio of the sound signal S2 that is output from the stethoscope 10 can be stably made to 60 dB or more. That is, with the protective layer 40 provided in which the acoustic impedance is adjusted to the acoustic impedance between the living body 12 and the piezoelectric film 30, sound reflection can be restrained and a sound can be collected with a high SN ratio.

It is preferable that any one of an elastomer material, a silicone resin, a silicone rubber, a urethane rubber, a natural rubber, a styrene-butadiene rubber, a chloroprene rubber, an acrylonitrile rubber, a butyl rubber, an ethylene propylene rubber, a fluoro-rubber, or a chlorosulfonated polyethylene rubber may be used as the protective layer 40. Since the protective layer 40 is directly pressed against the living body 12, it is desired that the material of the protective layer 40 is a material that does not cause discomfort caused by coldness or hardness to the living body 12. With the above-described material used, it is possible to restrain the occurrence of discomfort even in a case where the protective layer 40 is pressed against the living body 12.

Since the protective layer 40 is directly pressed against the living body 12, it is desired to have abrasion resistance. Therefore, it is preferable that the protective layer 40 has a measured value of 50 or more and 100 or less in a hardness test using a type A durometer conforming to ASTM D2240. A hardness measured by a durometer of another standard or another measuring method may be used to obtain the same hardness as the hardness in the above-described test method. With the protective layer 40 made to have a hardness in the above-described range, appropriate abrasion resistance for use as a stethoscope can be obtained.

In a case where the protective layer 40 has high stiffness, the expansion and contraction of the piezoelectric layer 32 is restricted and the vibration of the piezoelectric film 30 becomes small. Therefore, the thickness of the protective layer 40 having the above-described material and hardness may be appropriately set according to performance, handleability, mechanical strength, and the like required for the piezoelectric film 30. Specifically, the thickness need only be about 500 µm.

A surface of the protective layer 40, which comes into contact with the living body 12, is preferably roughened. Since the protective layer 40 is directly pressed against the living body 12, it is desirable that the protective layer 40 is easily peeled off from the skin of the living body 12. The roughening treatment method is not particularly limited, and various known methods such as mechanical roughening treatment, electrochemical roughening treatment, and chemical roughening treatment may be used. As a roughness for roughening the surface of the protective layer 40, which comes into contact with the living body 12, an arithmetic average roughness Ra is preferably 0.1 µm or more and 100 µm or less, and more preferably 0.1 µm or more and 10 µm or less. With the protective layer 40 made to have the roughness in the above-described range, the protective layer 40 can be easily peeled off from the skin of the living body 12.

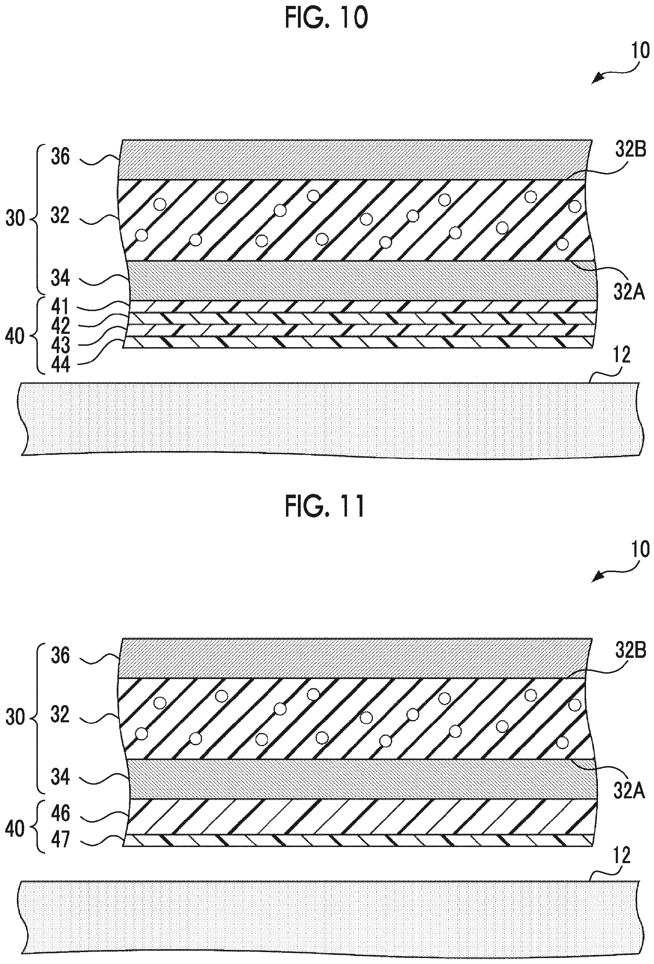

The protective layer 40 may be a layer of which the acoustic impedance is graded so that the acoustic impedance becomes lower toward the side that comes into contact with the living body 12. Alternatively, the protective layer 40 may be formed of a plurality of layers that are laminated so that the acoustic impedance becomes lower toward a layer on the side that comes into contact with the living body 12. For example, as shown in Fig. 10, in a case where the protective layer 40 is formed of four layers, the acoustic impedance need only become lowered in the order of the protective layers 41, 42, 43, and 44. In this case, the materials of the plurality of layers may be different from each other, or a material consisting of at least one of the above-described materials may be used. With the protective layer 40 made to have such a configuration, the difference in acoustic impedance between adjacent substances can be made smaller, and a sound can be collected with a higher SN ratio.

Further, the surface of the protective layer 40, which comes into contact with the living body 12, may consist of a silicone resin, and the silicone resin may have a siloxane skeleton as a main chain skeleton and at least one of a methyl group, a vinyl methyl group, or a phenyl methyl group, as a hydrophobic side chain. The surface of the living body 12 may be wet due to sweat or the like. Since the protective layer 40 is directly pressed against the living body 12, it is desired that at least the surface that comes into contact with the living body 12 is made of a material that is not denatured by moisture. Therefore, for example, as shown in Fig. 11, the protective layer 40 may be formed of a protective layer 46 of which the acoustic impedance is adjusted, and a protective layer 47 which is a surface that comes into contact with the living body 12 and which consists of a hydrophobic material. With the surface having hydrophobicity, which comes into contact with the living body 12, it is possible to restrain the protective layer 40 and the piezoelectric film 30 from being denatured even in a case where the protective layer 40 is pressed against the wet living body 12.

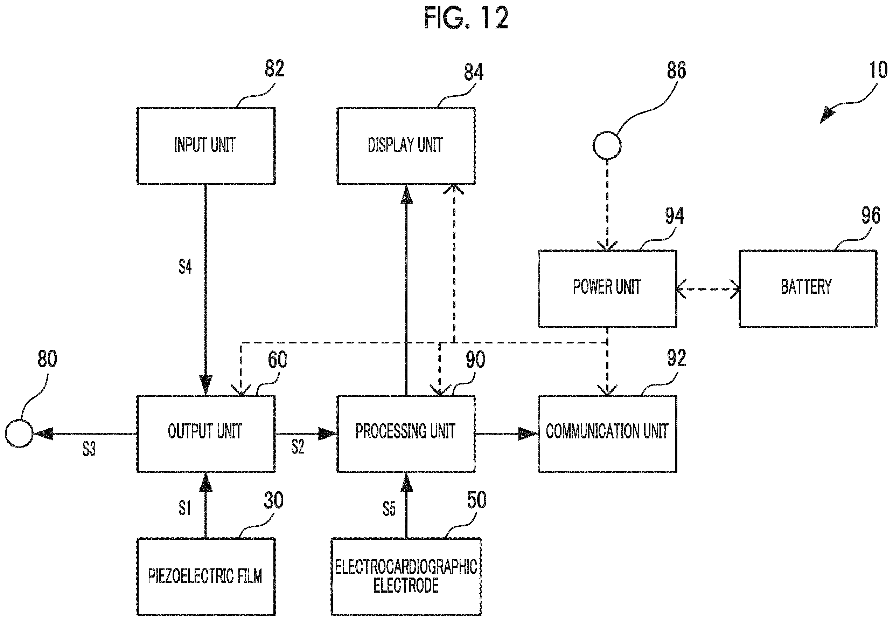

Next, with reference to Fig. 12, the function of the stethoscope 10 according to the present exemplary embodiment will be described. As shown in Fig. 12, the stethoscope 10 comprises the piezoelectric film 30, the electrocardiographic electrode 50, the output terminal 80, an input unit 82, the display unit 84, and the charging terminal 86. Further, the stethoscope 10 comprises an output unit 60, a processing unit 90, a communication unit 92, a power unit 94, and a battery 96. The output unit 60, the processing unit 90, the communication unit 92, the power unit 94, and the battery 96 may be housed in a housing portion 29, which is a space of the support base 20 (see Fig. 4), and may be provided outside the support base 20.

The piezoelectric film 30 detects the vibration caused by the body sound which is generated from the living body 12, and outputs a vibration signal S1 to the output unit 60 on the basis of the vibration. Specifically, in a case where a surface of the living body 12 is vibrated due to the body sound that is generated from the living body 12, when the living body 12 is brought into contact with the piezoelectric film 30, the piezoelectric film 30 is also vibrated in response to the vibration. The piezoelectric film 30 detects the vibration as a voltage that is generated between the first electrode 34 and the second electrode 36, and outputs the detected voltage to the output unit 60 as the vibration signal S1.

The input unit 82 receives an adjustment input of a level of the sound signal S2 that is output from the output unit 60, which will be described later, and outputs an adjustment input signal S4 indicating the received adjustment input information, to the output unit 60.

The output unit 60 outputs the sound signal S2 to the processing unit 90 on the basis of the vibration signal S1. Further, the output unit 60 adjusts the level of the sound signal S2 according to a level change of the vibration signal S1 and the adjustment input signal S4, and outputs the adjusted signal to the output terminal 80 as the adjustment signal S3. The output terminal 80 outputs the adjustment signal S3 to the outside.

The electrocardiographic electrode 50 detects the cardiac electrical activity of the living body 12. Specifically, in a case where the electrocardiographic electrode 50 is brought into contact with the vicinity of the heart of the living body 12, the electrocardiographic electrode 50 detects potential on a body surface of the living body 12 and outputs the detected potential to the processing unit 90 as an electrocardiographic signal S5.

The processing unit 90 performs predetermined processing on data based on the sound signal S2 and the electrocardiographic signal S5, and outputs the processed data to the communication unit 92. The processing unit 90 may comprise an amplifier circuit, a filter circuit, and the like, and may amplify the sound signal S2 and the electrocardiographic signal S5 or extract a specific frequency. Further, the data based on the sound signal S2 and the electrocardiographic signal S5 may be output as analog data or digital data. The processing unit 90 may be formed by, for example, a microcomputer including a central processing unit (CPU) 60, a read only memory (ROM), and a random access memory (RAM).

The communication unit 92 comprises a wired or wireless communication means, and transmits a body sound and electrocardiographic data to an external device. The communication means may be, for example, Bluetooth (registered trademark) or infrared communication, and the external device may be, for example, a personal computer or a smartphone.

The power unit 94 charges the battery 96 with electric power that is supplied from the charging terminal 86. Further, the power unit 94 supplies the electric power with which the battery 96 is charged to the output unit 60, the display unit 84, the processing unit 90, and the communication unit 92.

Further, the processing unit 90 acquires information indicating the remaining battery level of the battery 96 via the power unit 94, and may perform control, such as making LED light of the display unit 84 turned on or off, or blink on and off, on the basis of the acquired information. In this case, the user can grasp the remaining battery level of the battery 96 from a light emitting state of the LED light. Similarly, the processing unit 90, for example, may control the display unit 84 in a case where the sound signal S2 and the electrocardiographic signal S5 are normally acquired. In this case, the user can grasp an acquisition state of the sound signal S2 and the electrocardiographic signal S5 from a display mode of the display unit 84.

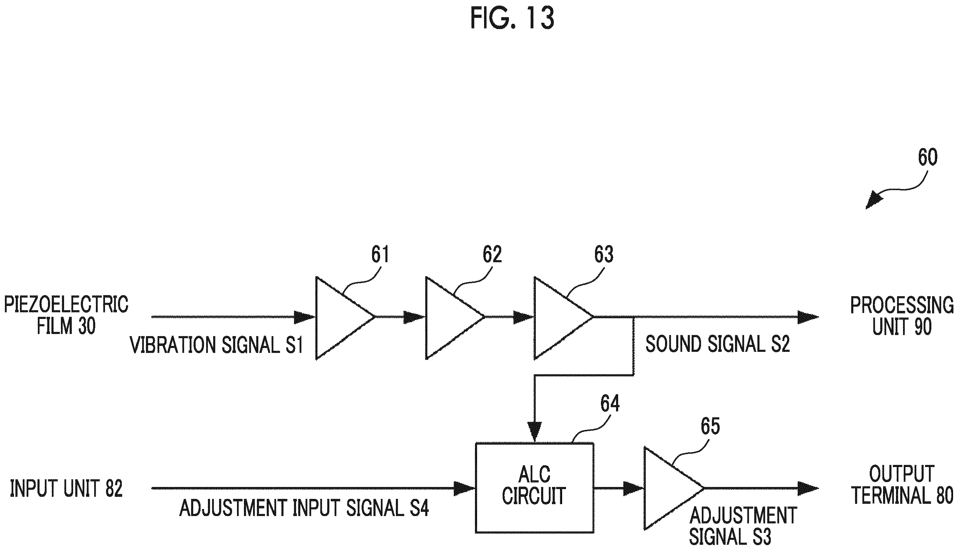

Next, the function of the output unit 60 will be described with reference to Fig. 13. As shown in Fig. 13, the output unit 60 comprises a buffer 61, a filter circuit 62, amplifiers 63 and 65, and an automatic level control (ALC) circuit 64.

The vibration signals S1 detected by the piezoelectric film 30 are input to the filter circuit 62 via the buffer 61 and noise is cut by the filter circuit 62, and then the signals are amplified by the amplifier 63 and output to the processing unit 90 and the ALC circuit 64 as the sound signal S2. The filter circuit 62 is, for example, a low-pass filter.

In a case where the stethoscope 10 is brought into contact with the living body 12 and then the stethoscope 10 is removed from the living body 12 after the auscultation, the piezoelectric film 30 is vibrated greatly, and sharp noise (spike noise) may be mixed into the vibration signal S1. In a case where the spike noise is output as the sound signal S2 from the output terminal 80 as it is, the sound heard from the earphone that is connected to the output terminal 80 becomes a harsh sound. Therefore, it is desirable to reduce the spike noise by the ALC circuit 64.

The adjustment input signal S4 from the input unit 82 and the sound signal S2 are input to the ALC circuit 64. The ALC circuit 64 may include, for example, a Schottky barrier diode that is used to detect spike noise from the sound signal S2, a capacitor that is used to smooth the sound signal S2, and a metal-oxide-semiconductor field-effect transistor (MOSFET) that is driven in a case where the spike noise is detected. The ALC circuit 64 uses the elements to reduce the spike noise and to adjust the level of the sound signal S2 on the basis of the adjustment input signal S4.

The sound signal S2 of which the spike noise is reduced and the level is adjusted by the ALC circuit 64 is amplified by the amplifier 65 and output to the output terminal 80 as the adjustment signal S3.

As described above, with the stethoscope 10 according to the present exemplary embodiment, the piezoelectric film 30 is supported by the support base 20 in a state in which the surface exposed from the opening portion 22 is convexly curved, and a protective layer 40 having an acoustic impedance between the acoustic impedance of the living body 12 and the acoustic impedance of the piezoelectric film 30 is provided on the surface of the piezoelectric film 30 on the side which comes into contact with the living body 12. Therefore, the body sound can be detected with a high SN ratio.

Further, with the stethoscope 10 according to the present exemplary embodiment, the output terminal 80 is disposed on the axis passing through the center of the one predetermined electrocardiographic electrode out of the three electrocardiographic electrodes 50, and the center of the piezoelectric film 30. Therefore, the operability of the user can be improved.

Further, with the stethoscope 10 according to the present exemplary embodiment, the elastic member 52 is a member having elasticity, which has an obtuse angle formed with the first contact surface 24 and which is connected to the first connection side 27 in the second connection side 57 having the same length as the first connection side 27 out of sides defining the second contact surface 54. Therefore, the electrocardiographic electrodes 50 can have a predetermined area while the increase in the size of the stethoscope 10 itself is restrained, and the three electrocardiographic electrodes 50 can be disposed sufficiently apart from each other. That is, it is possible to detect the cardiac electrical activity with a high SN ratio while the operability of the user is improved.

### [Second Exemplary Embodiment]

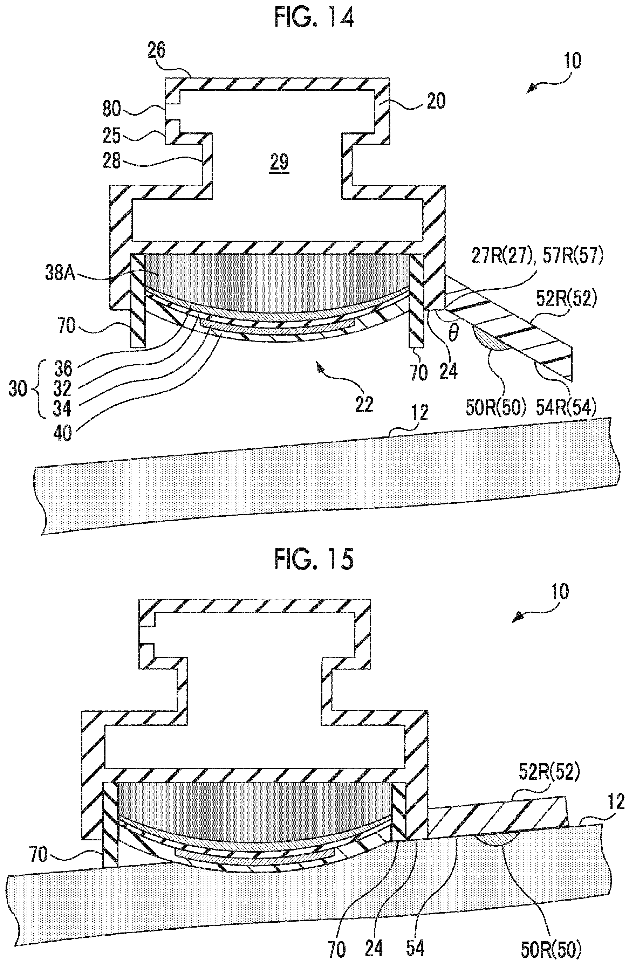

Next, the configuration of the stethoscope 10 according to the present exemplary embodiment will be described with reference to Figs. 14 and 15. In Figs. 14 and 15, the same reference numerals are assigned to the same elements as the elements described in the first exemplary embodiment, and detailed description thereof will not be repeated.

As in the stethoscope 10 according to the first exemplary embodiment, in a case where the stethoscope 10 that is supported by the support base 20 in a state in which the piezoelectric film 30 is convexly curved is pressed against the living body 12, a portion close to the outer periphery of the piezoelectric film 30 may be in a state of floating from the surface of the living body 12. For example, in a case where a patient who does not have specialized skills uses the stethoscope 10 in telemedicine or the like, it may happen that the stethoscope 10 cannot be pressed vertically against the living body 12, or the stethoscope 10 cannot be pressed against the living body 12 with a sufficient pressure. In a case where the piezoelectric film 30 is in a floating state, the piezoelectric film 30 can easily detect sounds from the outside, such as environmental sounds and human voices, (hereinafter, referred to as an external sound) as noise, and the SN ratio decreases.

Therefore, as shown in Fig. 14, the stethoscope 10 according to the present exemplary embodiment further comprises a sound insulating member 70 that surrounds the outer periphery of the portion of the piezoelectric film 30, which is exposed from the opening portion 22, and that blocks an external sound which is transmitted to the piezoelectric film 30, in addition to the configuration of the stethoscope 10 according to the first exemplary embodiment. Further, the tip of the sound insulating member 70 protrudes with respect to the convexly curved surface of the piezoelectric film 30. The protruding height of the sound insulating member 70 is preferably 1 mm or more.

In a case where the stethoscope 10 is pressed vertically against the living body 12, the sound insulating member 70 has elasticity enough to be compressively deformable so that the entire surface of the piezoelectric film 30, which is exposed from the opening portion 22, can be brought into contact with the living body 12. As the sound insulating member 70, a member having a high effect of absorbing an external sound, for example, a fiber-based material such as glass wool and a foaming material such as urethane foam is preferably used.

Fig. 15 shows a state in which the stethoscope 10 according to the present exemplary embodiment is pressed against the living body 12. In a case where the stethoscope 10 is pressed against the living body 12, the sound insulating member 70 is compressed in response to the pressure with which the stethoscope 10 is pressed. For example, as shown in Fig. 15, in a case where the stethoscope 10 is pressed diagonally against the living body 12, the degree of compression of the sound insulating member 70 is large in a place where the pressing force is large, and the degree of compression of the sound insulating member 70 is small in a place where the pressing force is small. With such a configuration, it is possible to restrain the piezoelectric film 30 from detecting an external sound even in a case where the stethoscope 10 cannot be pressed vertically against the living body 12 or the stethoscope 10 cannot be pressed against the living body 12 with a sufficient pressure.

As described above, with the stethoscope 10 according to the present exemplary embodiment, the sound insulating member 70 that surrounds the outer periphery of the portion of the piezoelectric film 30, which is exposed from the opening portion 22, and that blocks an external sound which is transmitted to the piezoelectric film 30 is further provided. Therefore, in a case where the stethoscope 10 is pressed against the living body 12, the piezoelectric film 30 can be surrounded by the sound insulating member 70, it is possible to restrain the piezoelectric film 30 from detecting an external sound as noise, and a body sound can be detected with a high SN ratio.

In the stethoscope 10 according to each of the above-described exemplary embodiments, the object to be measured is not limited to the living body 12, and the object to be measured may be a machine, a pipe, or the like. That is, detection of a sound that is generated from the machine, the pipe, or the like as the sound which is generated from the object to be measured can also be used to, for example, detect an abnormality of the machine or the pipe.

The disclosure of Japanese patent application 2019-138274 filed on July 26, 2019 is incorporated herein by reference in its entirety. Further, all documents, patent applications, and technical standards described in the present specification are incorporated herein by reference to the same extent as a case where each individual document, patent application, and technical standard are specifically and individually stated to be incorporated by reference.

## Claims

1. A stethoscope comprising:
a support base having a first contact surface that comes into contact with an object to be measured, in which each of three first connection sides that are at least parts of sides defining the first contact surface is at least a part of each side of a triangle including the first contact surface;
three elastic members having elasticity each of which has a second contact surface that has an obtuse angle formed with the first contact surface and that comes into contact with the object to be measured, the three elastic members being connected to each of the first connection sides in a second connection side having the same length as the first connection side out of sides defining the second contact surface; and
three electrocardiographic electrodes that are disposed on each of the second contact surfaces and detect cardiac electrical activity of the object to be measured.

2. The stethoscope according to claim 1, wherein:
the support base has an opening portion on the first contact surface, and
the stethoscope further comprises a detection unit that is supported by the support base in a state in which a part of a surface of the detection unit is exposed from the opening portion and that detects a vibration caused by a sound which is generated from the object to be measured.

3. The stethoscope according to claim 1 or 2, wherein each of the second contact surfaces has an area of 100 mm² or more.

4. The stethoscope according to any one of claims 1 to 3, wherein the elastic member has a thickness of 0.5 mm or more and 50 mm or less.

5. The stethoscope according to any one of claims 1 to 4, wherein the elastic member has a Young's modulus of 0.2 MPa or more and 50 MPa or less.

6. The stethoscope according to any one of claims 1 to 5, wherein the elastic member contains any one of an elastomer material, a silicone resin, a silicone rubber, a urethane rubber, a natural rubber, a styrene-butadiene rubber, a chloroprene rubber, an acrylonitrile rubber, a butyl rubber, an ethylene propylene rubber, a fluoro-rubber, or a chlorosulfonated polyethylene rubber.

7. The stethoscope according to any one of claims 1 to 6, wherein the support base is formed of a member harder than the elastic member.

8. The stethoscope according to any one of claims 1 to 7, wherein the elastic member has a shape that is tapered in a direction away from the second connection side.

9. The stethoscope according to any one of claims 1 to 8, wherein the first contact surface and each of the second contact surfaces are formed so as to extend in the same plane, in a case where a pressing force with which the first contact surface is pressed against the object to be measured is applied.

10. The stethoscope according to any one of claims 1 to 9, wherein the three electrocardiographic electrodes are a positive electrode, a negative electrode, and a reference electrode that measures a reference level.
